# EUROPEAN PATENT APPLICATION

(11) **EP 1 987 820 A1**
(43) Date of publication of application: **05.11.2008**
(21) Application number: 07009062.6
(22) Date of filing: 04.05.2007
(51) Int. Cl.: A61K 9/20, A61K 31/56, A61K 31/557

(54) **Slow release misoprostol for obstetric and/or gynaecological applications**

(71) Applicant: Fiala, Christian, Ph. D., 1060 Wien (AT); Gemzell, Kristina, Prof., Ph. D., 16775 Stockholm (SE)
(72) Inventor: Fiala, Christian, Ph. D., 1060 Wien (AT); Gemzell, Kristina, Prof., Ph. D., 16775 Stockholm (SE)
(74) Representative: Oser, Andreas

(57) **Abstract**

Described are medical applications of Misoprostol defined by a particular combination of sustained release or slow release dosage form and oral administration route for obstetric and/or gynaecological conditions and treatments. Further described are combination medications of Mifepristone with the aforementioned Misoprostol dosage form and administration.

## Description

The present invention relates to medical applications of Misoprostol defined by a particular combination of dosage form and administration route for obstetric and/or gynaecological conditions and treatments (in the following sometimes abbreviated as Ob/Gyn applications).

Misoprostol (15-deoxy-16-hydroxy-16-methyl PGE1; or (±) methyl 7-[3(α-hydroxy-2-β-(4(RS)-4-hydroxy-4-methyl-trans-1-octen-1-yl)-oxycyclopent-1α-yl]heptanoate; or (±) methyl methyl (13E)-11α,16-dihydroxy-16-methyl-9-oxo-13-prostenat) is a synthetic prostaglandin E1 (PGE1) analogue, developed because of its gastric acid anti-secretory properties and its various mucosal protective properties for the prevention and treatment of the ulcerogenic effects of non-steroidal anti-inflammatory drugs (Watkinson et al., Postgrad Med J 64, 60-77 (1988)). Because of its uterotonic and cervical priming action, it is contemplated in obstetric and gynaecological practice and for indications such as induced medical abortion, treatment of "missed" abortion or incomplete miscarriage and dilatation of the cervix prior to transcervical surgical interventions like placing an IUD, curettage in pregnant or non-pregnant women and surgical abortion. Misoprostol has been extensively studied in reproductive health (Goldberg et al., N Engl J Med 344, 38-47 (2001); Blanchard et al., Obstet Gynecol 99, 316-332 (2002)). Misoprostol is available in tablets containing 200 µg misoprostol (Cytotec^{®}, formerly commercialized by Searle, than Pfizer, and now by Pharmacia) produced and approved for oral use for the indication of gastric ulcer and for ameliorating drug-induced damage to stomach and gastric mucosa.

Misoprostol is also contemplated in combination with the progesterone receptor blocking agent Mifepristone (Mifegyne^{®}, Exelgyn, Paris, France; Mifeprex^{®} Danco, New York, US) for termination of early pregnancy and dilatation of the cervix prior to vacuum aspiration. For termination of early pregnancy, oral administration of misoprostol in combination with mifepristone is effective if the duration of amenorrhea is less than 50 days (World Health Organization 1997). Beyond this gestational age, clinical data indicate that oral misoprostol is less effective (Mc Kinley et al., Hum Reprod 8, 1502-1505 (1993)). In contrast, vaginal application is highly effective even after 49 days of amenorrhoea (El Refaey et al., N Eng J Med 332, 983-987 (1995)). Other routes of administration besides the oral one have therefore been studied, especially the vaginal and sublingual, as well as the buccal and rectal routes. They have also been used in obstetric and gynaecological applications.

There are only a few studies of the pharmacokinetic profiles of misoprostol in various administration routes. After a single oral dose, misoprostol is rapidly and almost completely absorbed from the gastrointestinal tract. However, the drug undergoes extensive and rapid first-pass metabolism (de-esterification) to form misoprostol acid (MPA), the principal active metabolite of the drug. Following 400 µg oral misoprostol, the plasma misoprostol level increases rapidly and peaks at about 30 minutes. However, the plasma level declines rapidly by 120 minutes after intake and remains low thereafter (Robert et al., Am J Dig Dis 12, 1073-1076 (1967); Zieman et al., Obstet Gynecol 90, 88-92 (1997); Tang et al., Hum Reprod 17, 332-336 (2002); Khan et al., Obstet Gynaecol 103, 866-870 (2004)). In contrast to the oral route, vaginal administration results in plasma concentrations that increase gradually, reaching a maximum level after 70-80 minutes and slowly declining, with detectable levels of MPA remaining 6 hours after administration (Zieman *et al., supra*). The peak concentration achieved is lower than following oral administration, but bioavailability measured as the area under the curve (AUC) of plasma MPA is significantly greater after vaginal administration. The greater bioavailability of vaginal misoprostol may help to explain why vaginal administration is more effective in inducing uterine contractions (Gemzell-Danielsson et al., Obstet Gynaecol 93, 275-280 (1999)).

An increasingly common alternative is to administer misoprostol sublingually. Pharmacokinetic studies (Tang *et al., supra*) show a rapid plasma peak level and a sustained elevation in MPA, resulting in a bioavailability that is higher than for the vaginal route. This results in the development of uterine contractility similar to that seen with vaginal treatment (Aronsson et al., Hum Reprod Jan 19, 81-84 (2004)). However, although vaginal absorption has been shown to be slower and the peak concentration achieved by the vaginal route is lower than that of the other routes of administration, the serum level of misoprostol is sustained for as long as, or possibly for longer than, with sublingual misoprostol. Furthermore, the effect on uterine contractility following sublingual administration seems to last for a shorter time than with vaginal administration (Tang *et al., supra,* Aronsson *et al., supra*). The effect of misoprostol may linger for more than 6 hours after a single dose administered vaginally.

A slow-release or sustained-release (SR) formulation of misoprostol and its *in vitro* dissolution rate into test medium was previously described, but for stabilization of misoprostol only and for its application in anti-culcer or antisecretory agent only (Chen et al., Int J Pharm 203,141-148 (2000); US 5,889,051, EP0896823). SR misoprostol has been studied with respect to short term pharmacokinetics and uterine contractibility (Fiala et al., Hum Reprod 20, 2648-2652 (2005a); Fiala et al., Hum Reprod 20, 3414-3418 (2005b)). Further experience with vaginally administered SR forms or suppositories of prostaglandins other than misoprostol, as studied with a SR-form of another PGE1 analogue (Bygdeman et al., Asia Oceania J. Obstet. Gynaecol. 10, 359-365 (1984); Smith and Baird, Br. J. Obset. Gynaecol. 87, 712-717 (1980)) and with a SR-form of another PGE2 analogue (Bygdeman et al., Contraception 22, 153-164 (1980) has been published.. However, the frequency of side-effects let the authors to abandon these other kind of prostaglandins for Ob/Gyn applications.

Various attempts have been made to address problems and complications or to improve the treatment or facilitating the regimen, by using various combinations of prostaglandins such as Misoprostol, and the progesterone blocking Mifepristone, including oral Mifepristone combined with vaginal Misoprostol for early medical abortion (Li et al., Taiwan J. Obstet. Gynecol. 45, 325-328 (2006); combination of oral Mifepristone followed by sublingual administration of Misoprostol (Lin et al., Taiwan J. Obstet. Gynecol. 45, 321-324 (2006)); Schaff et al., Contraception 59, 1-6 (1999); Schaff et al., Contraception 61, 41-46 (2000); Schaff et al., Contraception 64, 81-85 (2001); Schaff et al., Contraception 66, 247-250 (2002)); Mifepristone combined with Misoprostol administered sublingually or vaginally for medical abortion up to 13 weeks of gestation (Hamoda et al., BJOG 112, 1102-1108); or oral Mifepristone followed by vaginal Misoprostol 6 hours later or 36-48 hours later for termination of pregnancy in patients up to 63 days of gestation (Guest et al., BJOG 114, 207-215 (2007)). Previous medications however still proved to be unsatisfactorily effective and/or to be associated with a potential risk of adverse side-effects, which drawbacks have hampered to introduce relatively safe and widely accepted medication of prostaglandins such as Misoprostol alone or in combination with Mifepristone in Ob/Gyn applications. Trying to overcome these disadvantages with vaginal or sublingual application of Misoprostol let to other negative consequences and had the additional disadvantage of a lower preference by the patients.

The object of the present invention therefore was to provide an improved medication, which addresses the drawbacks of previous therapeutic approaches that hampered conventional obstetric and/or gynecological applications of prostaglandins, especially Misoprostol.

The object has been solved by providing a pharmaceutical formulation for medical obstetric and/or gynaecological treatment or for preparation of said treatment by oral administration, wherein the pharmaceutical formulation comprises methyl 7-[3(α-hydroxy-2-β-(4(RS)-4-hydroxy-4-methyl-trans-1-octen-1-yl)-oxycyclopent-1α-yl]heptanoate (Misoprostol) in a dosage form capable of sustained release of Misoprostol.

According to the present invention, it was surprisingly found, that an oral administration of a sustained release (also called slow release; here sometimes generally abbreviated by SR) dosage form of Misoprostol, i.e. the prostaglandin E₁(PGE₁) analogue (±) methyl 7-[3(α-hydroxy-2-β-(4(RS)-4-hydroxy-4-methyl-trans-1-octen-1-yl)-oxicyclopent-1α-yl]heptanoate, leads to a substantially longer effective blood level amount of its active metabolite, misoprostol acid (MPA), significantly longer than conventional oral administration of a normal (i.e. immediate release) dosage form, and substantially longer that a sublingually administered and a vaginally administered normal Misoprostol formulation. Even more surprisingly, the found substantially prolonged maintenance of the active metabolite, MPA, is achieved in spite of an even lower bioavailability (as determined by the area under the curve (AUC) of MPA plasma levels) compared with sublingually or vaginally administered dosage forms which had been previously preferred in therapeutic applications. Compared with other administration routes and dosage forms, the pharmaceutical formulation and administration according to the present invention allows for sufficiently strong onset of MPA plasma levels to effectively initiate uterine contractions, the MPA onset yet being moderate and smooth enough to reduced the risk of side-effects, while being combined with rhythmic uterine contractibility over an extended period of time. These findings prove to be particularly valuable for a range of obstetric and/or gynaecolocical applications, such as medical abortion at any time during gestation and in particular induced abortion in the period between the 49^{th} and 63^{rd} day after gestation; late abortion; missed abortion or incomplete spontaneous abortion or cervical priming prior to a transcervical surgical intervention, like insertion of an IUD, surgical abortion or hysteroscopy.

The present invention will now be described in further detail by referring to specific examples, embodiments and drawings, which however shall not be interpreted in a limiting way but are presented for illustration of the invention only. In the drawings:

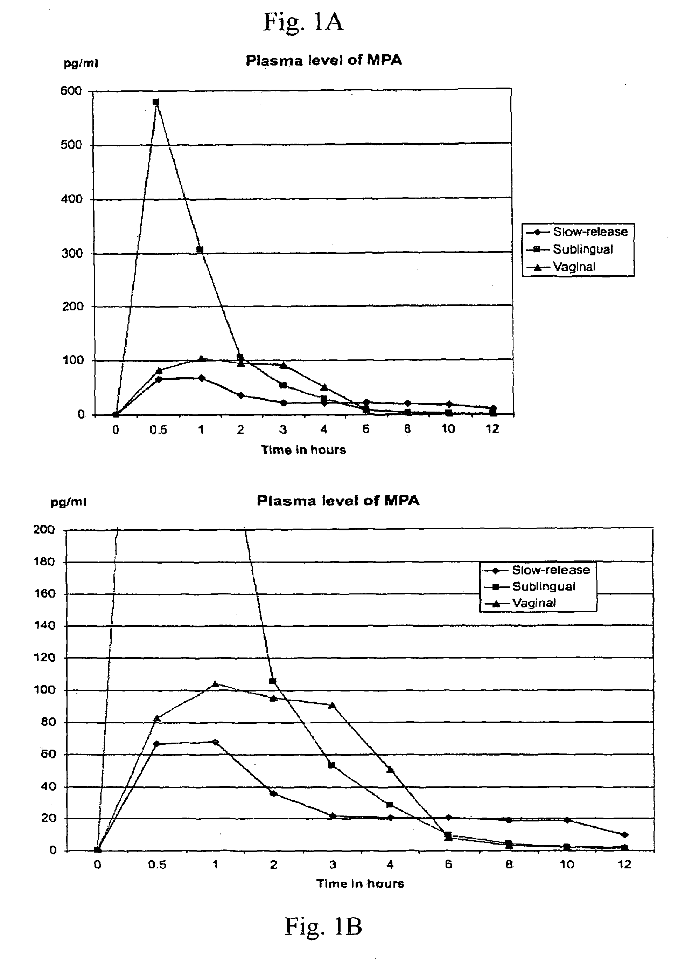

Figs 1A (overview) and 1B (detailed partial view) show a profile of the plasma level of MPA in pg/ml plasma *versa* time with respect to a pharmaceutical formulation of a sustained release dosage form of Misoprostol administered orally according to an embodiment of the present invention, compared to conventional administration routes and dosage forms (sublingually and vaginally);

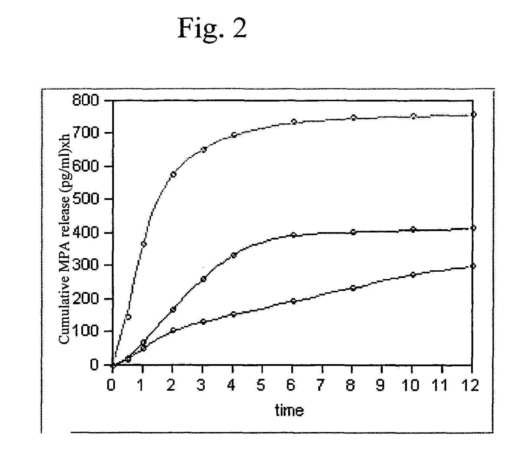

Fig. 2 shows a relationship between the "cumulative release of misoprostol acid" (MPA) (pg/ml) x h and time with respect to a pharmaceutical formulation of the present invention with a sustained release dosage form of Misoprostol administered orally, compared to that of conventional administration routes and dosage forms (sublingually and vaginally).

The dosage form capable of slow or sustained release (SR) of Misoprostol in the pharmaceutical formulation according to the present invention means a dosage form releasing Misoprostol longer than an immediate release. The Misoprostol SR dosage form suitable for oral administration thus releases Misoprostol substantially longer, and is thereby distinguished from conventionally orally administered Misoprostol. Oral administration according to the present invention has its normal meaning of administration into oral cavity, different from a sublingual administration.

The SR dosage form can be prepared by forming a semi-solid or solid mixture, dispersion, or entrapment of Misoprostol with a suitable polymer matrix. Core/shell structures or multi-layered structures suitable for sustained release are also possible. A suitable polymer matrix typically is a polymer which swells, or provides hydrogels in aqueous solutions in an appropriate pH range between 1.0 and 7.0, and which is insoluble or hardly soluble in aqueous solutions, for example based on acrylate or methacrylate polymers or copolymers; collagen or chitin gel systems; multi-layered polysaccharide matrix tablets; methyl cellulose derivatives such as hydroxypropyl-methyl-cellulose (HPMC), ethyl cellulose; natural or modified natural gums; PMMA grafted polysaccharides; wax-matrices prepared e.g. from Carnauba wax, bees wax, stearic acid, cethyl alcohol, cetosteryl alcohol, Kollidon SR and glyceryl monostereate alone or in combination as rate-retarding agents (which can be mixed by direct compression), biodegradable polymers such as poly(ortho)esters, etc. Also suitable is the use of a thermosensitive matrix polymer, for example [poly(ethylene glycol)-poly[lactic acid-co-glycolic acid]-poly(ethylene glycol)) (PEG-PLGA-PEG).
The ratio between Misoprostol and the matrix polymer may lie in a range of 1 part to about 1 - 1,000 parts. SR misoprostol suitable for oral administration can be produced by known techniques, as described for example by Chen et al. (Int. J. Pharm. 203, 141-148 (2000); US 5,889,051; EP0896823), Tyagi et al. (Pharm. Res. 21,832-837 (2004)), Varshosaz et al. (Drug Delivery 13, 295-302 (2006)), Bologna et al. (US 6,248,358), and others. A particularly well suited Misoprostol SR dosage from has been found to be defined by the particulars as described by Chen et al. (*supra*), i.e. forming solid dispersions of Misoprostol with methacrylate copolymers which are known as Eudragit, in particular Eudragit RS series and Eudagit RL series. Eudragit RLPO and RSPO alone or in admixture may be especially used.

Misoprostol can be suitably mixed with, or embedded within the above described polymer matrix by methods known to the person skilled in the art for preparing sustained release. Suitable methods include but are not limited to firstly dissolving misoprostol in a solvent like ethanol, methanol, methylenechloride or chloroform, then adding the matrix polymer to the solution and stirring to provide a mixed solution, followed by evaporating the solvent to obtain a solid dispersion, which can then be dried, grinded and sieved to yield the SR dosage form. Alternatively, the mixed solution may be spray-dried to prepare the solid dispersion in the form of granules, powder, capsules, pellets or the like for oral preparation. Optionally, stabilizers, emulsifiers, solubilisers, fillers, buffering agents or other auxiliary agents may be added to the prepared mixed solution. Additional or alternative methods to produce a SR dosage form include coating a misoprostol-containing core particle by one or more polymer layers capable of controlling the release of misoprostol from the core. Alternatively, an oral SR pharmaceutical formulation of misoprostol contains (a) an inert core which may be made of starch, a mixture of sugar and starch, or microcrystalline cellulose; (b) a dried emulsion comprising an effective amount of misoprostol deposited on the inert core, optionally further comprising a surfactant such as proloxamer and a solvent such as water, ethanol or glycerol; and (c) a coating deposited on top of the dried misoprostol emulsion coating, wherein the coating comprises at least one film-forming polymer insoluble or hardly soluble in aqueous solutions based on, e.g., hydroxypropyl methylcellulose (HPMC), hydroxypropyl cellulose (HPC), hydroxymethylcellulose (HMC), hydroxypropylmethyl-cellulose phthalate (HPMCP), cellulose acetate phthalate (CAP), co-polymerized methacrylic acid/methacrylic acid methyl esters, and optionally a plasticizer, alone or in combination. The pharmaceutical granules can be encapsulated. The granules can also be compressed into tablets by mixing the granule with at least one excipient which is e.g. selected from the group consisting of lactose, starch, talc, microcrystalline cellulose, and polyethylene glycol (PEG).
Other additional or alternative methods to produce a SR dosage form include incorporating misoprostol into biodegradable polymers.

The (oral) SR misoprostol may contain further ingredients, besides the aforementioned polymer matrix, core/shell structures or multi-layered structures mainly responsible for the sustained or slow release of the active agent. Such a further constituent may be generally selected from suitable pharmaceutically acceptable carriers or excipients. When misoprostol shall be combined with other active agents, especially with mifepristone as described further below, both active agents may be included in a common administration form, and the selected polymer matrix, the carrier or the excipient and other constituents of the formulation should be compatible with both active agents. If separate formulations are preferred for simultaneous or sequential use of both, misoprostol and the further drug and particularly mifepristone, the carriers, excipients or other constituents of the formulations do not need to be compatible and can be selected for each active agent, respectively. In the case of separate but combined use, also the optimal administration form combinations for the respective active drugs is selectable, i.e. the oral SR misoprosol combined with other forms for the further active agent and especially for mifepristone like orally, topically, vaginally, by injection, by infusion, by inhalation, by bolus, etc., although the present invention is not limited thereto. Besides a common sustained-release dosage form, a separate formulation for the optional further active agent such as mifepristone may be designed as solid, semi-solid, gelatinous or hydrogel-like, a liquid, a suspension, an emulsion, a powder, an ointment, a cream, an oil, a tablet, a pill, a capsule, a suppository and the like, or even a separate sustained-release dosage form. Suitable preparation processes for oral forms may, for example, include mixing, granulating, coating such as sugar-coating or other film-coating, tabletting, and the like.

Examples of further ingredients for either the oral SR misoprostol formulation, or for the common or separate formulation of the further active agent such as mifepristone include, but are not limted to diluents such as cellulose, starch, dextrose, saccharose, lactose; lubricants such magnesium or calcium stearate, stearic acid, talc, silica, polyethylene glycols; binding agents such as starch, methyl cellulose, caboxymethyl cellulose or other cellulose types, gelatine and polyvinyl pyrrolidone; disintegrating agents such as alginic acid, algenates, sodium starch glycolate; wetting agents such as polysorbates such as polysorbate 20 or polysorbate 80, lauryl sulfates, lecithin; coloring agents; sweeteners; aroma substances; carriers such as mannitol, sorbitol, saccharose; glycerol; polyvinyl alcohol; cellulose types; oils; glycols such as propylene glycol; oleate; fatty stubstances and fatty-like substances, lipoids, phospholipids, hydrocarbons; preservatives; amino acids such as glycine; urea; buffering agents such as phosphates; sodium chloride; and the like.

A suitable sustained release can be obtained, when the SR dosage form of Misoprostol prepared from the aforementioned polymer matrices or other SR structures, which optionally include further ingredients, shows a dissolution such that, for example, half of the incorporated Misoprostol dissolves over a time period of at least 5 hours, more preferably of at least 10 hours, and even more preferred of at least 12 hours, when tested in an aqueous test solution, preferably containing a surfactant at a concentration of 0.05% to 0.3 %, in a pH range between 1.0 and 7.0 at 37°C, by using a USP paddle dissolution tester (suitably with an agitation speed of 75 rpm). Particularly preferred SR dosage forms are those which can achieve the preferred MPA plasma profile parameters described in the following.

According to the present invention, it has been surprisingly found that, albeit a very short biological half-life of Misoprostol once released within the body subsequent to administration, the Misoprostol SR dosage forms when orally administered are associated with optimized MPA plasma profiles reaching up to the period of at least 10 hours and even at least 12 hours. Moreover, the SR dosage form of Misoprostol when orally administered unexpectedly displays, in striking contrast to a sublingual and a vaginal administration route, an extended increase of cumulative release of the active metabolized agent in blood (determined as an amount of MPA per ml blood per hour) extending longer than 6 hours and even longer than 10 hours. More beneficially, the cumulative release increase shows a linear or quasi linear increase behaviour over the aforementioned time periods, contrary to other administration route/dosage form combinations. Moreover, the SR dosage form of Misoprostol used according to the present invention by oral administration can provide the above-described prolonged activity in blood, while beneficially keeping a maximum plasma level of the active analogue MPA below 100 pg/ml, more beneficially below 80 pg/ml blood plasma, but suitably with a maximum plasma level of at least 10 pg/ml, more preferably at least 20 pg/ml and particularly at least 50 pg/ml blood plasma. Further, the extended plateau level after maximum, preferably maintained for a prolonged time of at least 4 hours and more preferably 6 hours, can be made to hold a level of at least 10 pg/ml blood plasma and more preferably at least 15 pg/ml blood plasma, up to a total time period from administration of 10 hours or longer. The aforementioned parameters are particularly valuable for a number of useful applications described in further detail below.

The amount of Misoprostol contained in the pharmaceutical formulation of the present invention shall be appropriate to be effective in the treatment of the Ob/Gyn condition, or for preparing the desired treatment. The amount of Misoprostol in the total pharmaceutical formulation is preferably higher than 200 mcg (µg), more preferably 400 mcg or higher, and can be made up to 5mg or even higher depending on the polymer matrix or other SR structure design. A preferred range is 800 mcg to 1,600 mcg Misoprostol with respect to the total pharmaceutical formulation. Compared with previously practised immediate release dosage forms of Misoprostol, which typically required a repeated administration of Misoprostol per medical treatment regime, the present invention enables a single dosis application which is much in favour of convenience of the female patients. When desired or considered necessary, of course an oral administration 2x, 3x or multiple times of a dosage form can be used. However if repeated administration might be necessary, the intervals can be considerably longer than with conventional misoprostol.

The unique properties and results found to be associated with the SR dosage form of Misoprostol when orally administered according to the present invention enables valuable medical treatments of a female patient. The properties and results can open therapeutic windows, which are particularly effective for the obstetric or gynecological indications, especially those where induced abortion in the period between the 49^{th} and 73^{rd} day of gestation or later; late abortion (especially based on a medical indication); missed abortion involving dead foetuses (intrauterine fruit death, IUFD); or incomplete spontaneous abortion is treated, A particularly valuable application is a single administration of SR Misoprostol orally for cervical priming prior to surgical treatment such as curettage for abortion, vacuum aspiration, IUD insertion, hysteroscopy etc.. Here, the pharmaceutical formulation and administration form according to the present invention enables a convenient and advantageous priming: Instead of a conventional timing of oral or vaginal administration 3 to 4 hours on the same day prior to the intervention or surgical treatment, the pharmaceutical formulation according to the present invention now allows the active drug to be given in the evening before the intervention or surgical treatment, while providing a good priming effect on the next morning. Further, independent from an over-night timing scheme, the present invention provides an extended timing freedom, as the exact time of the surgical intervention is less important owing to the very long plasma levels of the active agent.

The present invention moreover provides an improved combination of the aforementioned oral SR dosage form of Misoprostol with another active agent, Mifepristone (11β-[p-(dimethylamino)phenyl]-17β-hydroxy-17-(1-proynyl)estra-4,9-dien-3-one), in a combination medication. The pharmaceutical approach for the combination medication includes methods for separate, for simultaneous or for sequential use in the preparation of treatment, or in the treatment of an obstetric and/or gynaecological condition according to the needs and appropriate administration timings. Again, the unique properties of the SR dosage form of Misoprostol according to the present invention as described above allow for particularly beneficial combinations with Mifepristone, in particular those as described in the following.

The findings of the present invention have the potential to improve the treatment in a number of clinical situations. Generally, it can be stated that the invention is applicable in OB/Gyn applications, in reproductive medicine, etc. It is especially useful in cases were sufficiently strong onset as well as prolonged uterine contractions are needed or are of particular benefit. Some of them are described in more detail in the following:
1. Giving orally SR misoprostol at any gestational age
   Owing to the surprising results obtained by the oral administration of SR misoprostol, there appears in principle no limitation to the gestation status of the women to be treated. There would be no need to diagnose the exact pregnancy length and apply different regimens. Further, SR misoprostol can be preferably given orally at the same time as mifepristone in medical abortion of any gestational age.
2. Giving mifepristone followed by an orally SR misoprostol after a time interval of 6 to 48 hours for medical abortion (especially in gestations later than 7 weeks gestation) (counted from the 1 st day of last menstrual bleeding)
   Conventionally, mifepristone followed by misoprostol given orally is the approved regimen up to 49 days of gestation. However the oral application of normal misoprostol after pretreatment with mifepristone in gestations beyond 49 days has proven to be of insufficient efficacy. The prostaglandin therefore had conventionally to be given vaginally. Consequently all evidence based guidelines (RCOG in the UK, ANAES in France, WHO, etcs) recommend vaginal application of misoprostol in medical abortion, especially in gestations beyond 49 days LMP. (It should be mentioned that normal Misoprostol is approved for oral application only. Vaginal application is therefore "off-label". Furthermore there have been some death cases reported in the US in women who had administered misoprostol vaginally. Consequently vaginal application of misoprostol is not recommended by some health care providers in the US (Planned Parenthood Federation of America).
   Aside the improved effects associated with the present invention, oral SR misoprostol provides several additional advantages for the medical treatment:
   - most women prefer oral administration of a drug to the vaginal route
   - currently no alternative to vaginal misoprostol exists in gestations above 49 days, although a debate has taken place following severe problems which occurred after vaginal adminstration of misoprostol.
3. Giving slow release misoprostol orally for cervical priming
   Giving conventional misoprostol for cervical priming prior to a curettage for abortion, IUD insertion etc. is well established and becoming increasingly popular. The recommendations are mainly oral or vaginal administration 3-4 hours prior to the intervention. The inconvenience of current regimes is this delay as it is difficult to administer. And giving the conventional misoprostol on the evening before the intervention is too far away and leads to a reduced effect.
   Here, the oral SR misoprostol administration could be beneficial. It could be given the evening before the intervention and would most probably have a good priming effect on the next morning. Also the exact time of the surgical intervention would be less important as plasma level of the SR misoprostol is very long.

### Examples

### Example 1

### Outline and setup of gynaecological treatment study of misoprostol-containing formulations

A study was performed with healthy women ≥18 years with a viable intrauterine pregnancy of up to 12 weeks (47-83days) of gestation who had requested termination of pregnancy by vacuum aspiration.

After giving their informed consent, eligible women were allocated randomly to the three treatment groups using computer generated random tables and numbered, sealed, opaque envelopes opened consecutively. The participants were allocated to receive either 2 tablets misoprostol (Cytotec® 200 µg) administered sublingually (9 patients) or vaginally (10 patients), or 2 tablets SR misoprostol (400 µg/ tablet), i.e. a total of 800 µg single dose administered orally (11 patients). SR misoprostol had been prepared by dispersing misoprostol in ammonio methacrylate copolymer (Eudragit) according to Chen et al. (Int. J. Pharm. 203, 141-148 (2000)). The SR misoprostol tablets were swallowed with a small amount of water. Women allocated to vaginal misoprostol treatment inserted the tablets intravaginally themselves.

The women were admitted to hospital in the morning after fasting overnight. Following the insertion of an intravenous 18-gauge catheter suitable for repeated blood-sampling, preferably into the antecubital vein, a baseline blood sample was obtained and the envelope with the treatment dosage and administration route was opened.

Venous blood samples (10 ml) were taken before treatment (0 or baseline test) and up to 12 hours after treatment at 0.5, 1, 2, 3, 4, 6, 8, 10 and 12 hours after administration of misoprostol. The blood samples were immediately cooled in a refrigerator at +6°C for 30-60 minutes and thereafter centrifuged in a cooled centrifuge for 10 minutes at 3000 rpm. The plasma was then drawn from the blood samples and stored at below -20°C for further analyses. Vacuum aspiration was performed according to clinical routine 3 hours after misoprostol treatment.

Based on a former published GC-MS/MS method (Watzer et al., J Mass Spectrom 37, 927-933 (2005), MPA was measured in plasma samples using a modified LC-MS/MS isotope dilution assay. After addition of 15(S)-15-methyl prostaglandin E2 (15-methyl-PGE2) as internal standard, MPA was extracted using a monolithic reversed phase cartridge. After consecutive clean-ups, the prostanoids were eluted with diisopropyl ether. The dried and reconstituted sample was determined by LC-MS/MS using the molecular ions [M]- as precursor in the negative ion electrospray ionization (ESI) mode. The product ions used for quantification were [M-2H2O-C6H10-H]- (MPA) and [M-2H2O-CO2-C7H12-H]- (15-methyl-PGE2), respectively. The extraction recovery for MPA was about 95%. The limit of detection was 1 pg/ml MPA in serum samples. The correlation coefficients of the linear calibration graphs for MPA were r > 0.998 in the 10-1000 pg/ml range for the tested matrix. For the spiked quality control standards of MPA, the inter-day precision ranged from 4.3% to 9.7%, with an inter-day accuracy (relative error) between -8.7% and 7.2%. The intra-day precision and relative error of MPA ranged from 4.2% to 6.2% and between -7.3% and 6.9%. The detection limit of assay was 1 pg/sample.

The MPA plasma concentration profile, the area under the curve (AUC) of the MPA concentration profile up to 720 minutes after administration of misoprostol, the peak concentration of MPA (Cmax) and the time to peak concentration (Tmax) were determined. AUC was calculated according to the trapezoidal method, by dividing the area under the curve into trapezium segments according to the time intervals of blood sampling. The area of each segment was computed according to the following formula for the calculation of trapezium area: 0.5 [Cx + Cx-1] [ time interval ], x= 1 - 10. The AUC₇₂₀ was calculated by summation of the trapezium segments. Pulse, blood pressure and temperature were measured repeatedly and side-effects such as nausea, abdominal pain, skin rash or diarrhoea were recorded, as well as any analgesics given.

Kruskal-Wallis tests were used to test the overall difference between the three groups. If the overall difference was significant, Wilcoxon two-sample tests were used to test pair-wise differences. P < 0.05 (two-tailed) was considered statistically significant. P-values were not corrected for multiple comparisons. However, all stated differences remained significant (P < 0.05) after applying Bonferroni-Holm correction (Holm, Scand J Stat 6, 65-70 (1979)). Statistical analyses were performed by PROC NPAR1WAY (exact tests) in SAS/STATTM software (SAS Institute Inc, Cary, NC (1997)).

### Results

The clinical characteristics of the patients are shown in Table I. Women in the three groups did not differ significantly in terms of age, number of pregnancies, parity, gestational age, height, weight or body mass index (BMI). Gestational length for all women was 47 to 83 days and the median number of pregnancies was 3 (range 1-8), while parity was 0 (range 0-5).

**Table I. Pharmacokinetic parameters of the three routes of misoprostol administration**

| | | | | |
|---|---|---|---|---|
| Misoprostol | 800 □g SR | 400 □g sublingual | 400 □g vaginal | p^{a} |
| Route | n=11 | n=9 | n=10 | |
| | | | | |

| Peak concentration of misoprostol acid (Cmax) (pg/ml) | | | | |
|---|---|---|---|---|
| Mean±SD | 78.8±51.1 | 580±178.1 | 117.7±42.1 | p<0.0001^{b} |
| CV | 64.8 | 30.7 | 35.8 | |
| | | | | |

| Time to attain peak concentration (Tmax) (min) | | | | |
|---|---|---|---|---|
| Mean±SD | 1.6±2.8 | 0.5±0 | 1.7±1.2 | p= 0.0094^{c} |
| CV | 175 | 0 | 70.6 | |
| | | | | |

| Area under the curve to 720 min (AUC₇₂₀) (pg h/ml) | | | | |
|---|---|---|---|---|
| Mean±SD | 301.5±105.8 | 757.8±200.5 | 414.6±128.2 | p<0.0001^{d} |
| CV | 35.8 | 26.5 | 30.9 | |

| | | | | |
|---|---|---|---|---|
| a. Kruskal-Wallis test. Overall comparison between the three groups. b. Cmax was significantly higher in the 400 µg sublingual group than in the 800 µg SR and the 400 µg vaginal group respectively (p<0.0001). In the vaginal group, it was significantly higher than in the SR group (p = 0.0242). c. Tmax was significantly shorter for the 400 µg vaginal group compared to the 800 µg SR group (p= 0.0379) and for the 400 µg vaginal group (p= 0.0027) respectively. There was no significant difference between the SR and vaginal groups. d. The AUC₇₂₀ was significantly greater for the 400 µg sublingual group compared to the 800 µg SR and 400 µg vaginal groups (p<0.0001) respectively. The AUC₇₂₀ was significantly greater in the vaginal group than in the SR group (p = 0.043). b-d. Wilcoxon two-sample test CV coefficient of variation (%) | | | | |

The mean serum concentration profiles over time of MPA after administration of 400 µg sublingual or vaginal misoprostol or 800 µg oral SR misoprostol are shown in Figure 1A (overview) and Fig. 1B (detailed view). A number of pharmacokinetic parameters were studied, namely the peak concentration (Cmax), time to peak concentration (Tmax) and AUC₇₂₀ (Table I). The individual variability of these pharmacokinetic parameters was denoted by the coefficient of variation (CV).

The maximum plasma level, Cmax, and time to peak concentration, Tmax, differed significantly between the three groups. Treatment with sublingually administered misoprostol resulted in the earliest serum peak concentration of MPA (Fig 1A, Table I). Sublingual administration also resulted in the highest maximum plasma level, Cmax, which was significantly higher than the peak levels following SR misoprostol or vaginal administration of misoprostol (p<0.0001). The time to peak plasma concentration of MPA was significantly shorter following sublingual administration, 0.5 hour, compared to SR administration (p = 0.0379) and vaginal administration (p = 0.0027). Treatment with vaginal and SR misoprostol resulted in plasma peaks of MPA 1.6 to 1.7 hours after administration.

Plasma levels following oral SR misoprostol administration remained significantly elevated, even for a period up to 12 hours. Especially in the period starting 5 to 6 hours after administration of misoprostol, plasma levels of MPA in the oral SR group significantly exceeded those in the other groups. Although bioavailability, measured as the AUC₇₂₀, was highest in the sublingual group and still high in the vaginal group but lowest following oral SR misoprostol, the cumulative AUC for the sublingual and vaginal groups did not continue to rise after 6 hours, contrary to that of the oral SR group (Fig 2).
In terms of overall efficacy, the duration of plasma levels above a certain threshold seems to be more important than the absolute level of MPA or the AUC. Oral treatment with misoprostol leads to a high but short-lived peak of MPA which does not generate uterine contractions. On the other hand, prolonged and rhythmic uterine contractions are seen after treatment with SR misoprostol despite lower serum levels of MPA and a lower AUC compared to vaginal misoprostol. The elevation of MPA also lasts longer.
On the other hand, the incidence of side-effects seems to be associated with high plasma levels of MPA. Conventional (immediately released) oral misoprostol results in a rapid and short lived plasma peak after intake. This is even more pronounced for the sublingual route. Oral and particularly sublingual misoprostol also lead to the highest reported incidence of side-effects.

The surprising effects and properties associated with an oral SR misoprostol administration could not have been expected or foreseen from short term short term pharmacokinetics and uterine contractibility (Fiala *et al.* (2005 a) *supra,* Fiala *et al.* (2005 b) *supra*). The new findings of the present invention are however associated with valuable medical treatments (which includes the concept of preparation of treatments) of obstetric and/or gynaecological conditions due to an early onset sufficiently strong to exceed a certain threshold for initiating uterine contractions, combined with a prolonged elevated plateau of active MPA level substantially long by exceeding 6 hours, and sufficiently high in order to maintain rhythmic uterine contractions. A certain level of MPA is needed, but once reached, the extended duration of elevated MPA seems to be the most important parameter. Prolonged stimulation of the uterus is significant to overcome the progesterone block which normally prevents uterus contractions (Csapo, Am J Anat 98, 273-291 (1956)).

The effects and properties associated with an oral SR misoprostol administration therefore opens the possibility for an oral administration of a single dose while nevertheless providing excellent efficacy, rather than a necessity for a repeated (2x, 3x or more times) oral or sublingual administration of conventional, immediately releasing misoprostol forms. Repeated use of the pharmaceutical formulation of the present invention is thus conceivable for cases where specially medically indicated. Further, oral SR dosage forms of misoprostol tends to have less potential risks and side-effects associated with other administration forms, as these problems appear to be mainly correlated with maximum levels of the active compound, MPA, reached after a certain administration form and route.
In summary, the present invention is particularly useful in cases were the effects found according to the present invention are effectively displayed, i.e. both a sufficiently strong onset of uterine contraction and a prolonged maintenance uterine contractions are beneficial, while on the other hand the maximum blood level is however still moderate enough to reduce adverse side-effects.

Further examples of oral formulations comprising SR Misoprostol are described in the following:

### Example 2

### (PEG-PLGA-PEG) matrix polymer for sustained release

A 0.02% w/v ethanol solution of misoprostol in a 30% w/v dispersion of thermosensitive polymer [poly(ethylene glycol)-poly[lactic acid-co-glycolic acid]-poly(ethylene glycol)) (PEG-PLGA-PEG) in a 0.1 M phosphate buffer is used to prepare a SR misoprostol formulation as described by Tyagi et al. (Pharm. Res. 21,832-837 (2004)), designed for use in oral administration.
A dosage of this formulation containing 600µg misoprostol can be taken orally in a single dose by women for cervical priming in the evening prior to surgical intervention.

### Example 3

### Eudragit RLPO and RSPO matrix polymer for sustained release

A solid dispersion of misoprostol for oral administration is produced by solvent method as described by Varshosaz et al. (Drug Delivery 13, 295 - 302 (2006)), containing 7% of misoprostol and a mixture of Eudragit RLPO and RSPO at a ratio of 5:5 and having a particle size of less than 100 µm.
A dosage of this formulation containing 800µg misoprostol can be taken orally in two doses separated by 1 day by women suffering from intrauterine foetal death.

### Example 4

### Ammonio methacrylate copolymer matrix for sustained release

A SR misopristol oral dosage form containing misoprostol is prepared as described by Example 10 of US 5889051 with the difference that 800 µg of the drug, misopristol, is incorporated into the oral formulation.

### Example 5

### Separate and simultaneous or sequential use for combination medications of oral SR Misoprostol and Mifepristone

A pharmaceutical combination preparation for separate and simultaneous or sequential modes of administration according to the invention can be made as follows.

A SR misopristol oral dosage form containing 800 µg misoprostol is prepared as described by Example 10 of US 5889051 (see Example 4 above).

A separate mifepristone-drug formulation for oral administration is prepared from the following ingredients:

| | |
|---|---|
| mifepristone | 200 g |
| lactose | 200 g |
| corn starch | 250 g |
| talc powder | 20 g |
| magnesium sterate | 5 g |

The components are suspended in 50 ml warm water, homogenously mixed and processed into 1,000 tablets. Each tablet obtained is suitable for oral administration of 200 mg mifepristone.
For induced abortion, eligible women in the status of the period between the 49^{th} and 63^{rd} day after gestation can receive a mifepristone 200 mg tablet orally, and a single dosis of SR misoprostol 800 µg orally either simultaneously, or subsequently 6 hours or 36-48 hours later.

### Example 6

### Common simultaneous use for combination medications of oral SR Misoprostol and Mifepristone

A pharmaceutical combination preparation for simultaneous mode of administration in a common oral SR formulation according to the invention can be made as follows.

A combined common SR misopristol/mifepristone oral dosage form is prepared as described by Example 10 of US 5889051 except that 400 µg (alternatively 600 µg, 800 µg or 1200 µg) of misoprostol and 200mg of mifepristone are incorporated.
The obtained combined common oral SR formulation can taken orally by eligible women in the status of the first trimester for medical abortion.

## Claims

1. Pharmaceutical formulation for medical obstetric and/or gynaecological treatment or for preparation of said treatment by oral administration, the pharmaceutical formulation comprising methyl 7-[3(α-hydroxy-2-β-(4(RS)-4-hydroxy-4-methyl-trans-1-octen-1-yl)-oxycyclopent-1α-yl]heptanoate (Misoprostol) in a dosage form capable of sustained release of Misoprostol.

2. The pharmaceutical formulation according to claim 1, wherein the dosage form is one which extends plasma or serum levels of Misoprostol acid, subsequent to oral administration, to a period of at least 10 hours.

3. The pharmaceutical formulation according to any one of the preceding claims, wherein the dosage form is one which provides an increase of cumulative amount of active agent in serum or plasma (amount per ml serum or plasma x h) extending longer than 6 hours.

4. The pharmaceutical formulation according to any one of the preceding claims, wherein the dosage form is one, which provides a maximum plasma level of Misoprostol acid to be in a range of between about 20 pg/ml and about 100 pg/ml serum or plasma.

5. The pharmaceutical formulation according to any one of the preceding claims, wherein a single oral dosis is used for medical treatment of a female patient.

6. The pharmaceutical formulation according to any one of the preceding claims, wherein induced abortion in the period between the 49^{th} and 63^{rd} day gestation; late abortion; missed abortion or incomplete spontaneous abortion is treated.

7. The pharmaceutical formulation according to any one of claims 1 to 5, wherein a patient is treated orally for cervical priming at least 6 hours prior to surgical operation.

8. Combination medication for separate, simultaneous or sequential use in the preparation of treatment, or in the treatment of an obstetric and/or gynaecological condition, said combined medication comprising as active agents, in a common or in separate pharmaceutically acceptable formulation(s):
• Mifepristone (11β-[p-(dimethylamino)phenyl]-17β-hydroxy-17-(1-proynyl)estra-4,9-dien-3-one); and
• Misoprostol (methyl 7-[3(α-hydroxy-2-β-(4(RS)-4-hydroxy-4-methyl-trans-1-octen-1-yl)-oxycyclopent-1α-yl]heptanoate) in a pharmaceutical formulation as set forth in any one of claims 1 to 4;
respectively in amounts effective to treat the desired obstetric and/or gynaecological condition.

9. Combination medication according to claims 8, wherein the Misoprostol-containing pharmaceutical formulation is given in a time interval of 6 to 48 hours subsequent to administration of a Mifepristone-containing pharmaceutical formulation for medical abortion.

10. The combination medication according to claim 8 or 9, wherein the combined administration scheme is started at least 7 weeks after gestation. -

11. The combination medication according to claim 8, wherein the pharmaceutical formulation according to any one of claims 1 to 4 is administered orally, simultaneously with an oral administration of Mifepristone.

12. The combination medication according to claim 11, wherein Mifepristone is included within the same dosage form as Misoprostol.
